# EUROPEAN PATENT SPECIFICATION

(11) **EP 3 721 904 B1**
(45) Date of publication and mention of the grant of the patent: **13.10.2021**
(21) Application number: 20174783.9
(22) Date of filing: 19.11.2010
(51) Int. Cl.: A61K 39/395, A61K 9/19, A61K 47/26

(54) **FORMULATIONS OF T1H ANTIBODY**
FORMULIERUNGEN VON T1H ANTIKÖRPERN
FORMULATIONS D'ANTICORPS T1H

(30) Priority: 20.11.2009 IN 2859CH2009
(43) Date of publication of application: 14.10.2020
(62) Divisional of application: 10831248.9
(73) Proprietor: Biocon Limited, Bangalore 560 100 Karnataka (IN)
(72) Inventor: RAMANI, Karthik, 560078 Bangalore (IN); JAYAKAR, Sucharitha, 560103 Bangalore (IN)
(74) Representative: HGF

(56) References cited:
- EP-A1- 2 119 453
- WO-A1-2008/071394
- US-B1- 6 267 958

## Description

### FIELD OF THE INVENTION

The present invention relates to stable formulations of antibody. A Preferred stable formulations comprise: 25 - 250 mg/ml antibody, 10 to 30 mM a buffering species, 1 to 15% polyol, 0.001 % to 0.05 % surfactant and pH from 5 to 7.5. A further aspect of the invention features the process of preparing the aforesaid formulations.

### BACKGROUND AND PRIOR ART OF THE INVENTION

Monoclonal antibodies (mAbs) have permitted the characterization of molecules of physiological importance expressed on the cell surface. Defined in the cells of the Immune System the "Leukocyte Differentiation Clusters" or antigens (CD) (scholossman, S. F. et al. (1994) Immunol. Today 15 (3);98). The definition of the role of the CD's in the differentiation and maturation of the lymphoid cells during their ontogenic development, in the mechanisms of cellular recognition and adhesion and in the mechanisms of activation and proliferation during the immune response have conducted to the use of their respective mAbs in diagnosis and immunotherapy, with promising results (Dantal, J. et al. (1991) Curr. Opin. Immunol. 3:740

Recent advances in the development of biotechnology have provided a wide variety of biologically active polypeptides in sufficiently large quantities for use as drugs. Polypeptides, however, can lose their potent biological activity as a result of physical instabilities, including denaturation and formation of soluble and insoluble aggregates, and a variety of chemical instabilities, such as hydrolysis, oxidation, and deamidation. Stability of polypeptides in liquid pharmaceutical formulations can be affected, for example, by factors such as pH, ionic strength, temperature, repeated cycles of freeze-thaw, and exposure to mechanical shear forces such as occur during processing. Aggregate formation and loss of biological activity can also occur as a result of physical agitation and interactions of polypeptide molecules in solution and at the liquid-air interfaces within storage vials.

US20030190316 relates to stabilized preparations containing an antibody in a glycine buffer and/or a histidine buffer and also provides processes for preparing a protein-containing stabilized preparation, comprising adjusting the pH with a basic amino acid or a basic amino acid derivative or a salt thereof.

While a number of liquid pharmaceutical compositions have been formulated to stabilize the biological activity of polypeptides contained therein, the degradation of polypeptides in liquid formulations continues to create problems for medical practitioners. Consequently, there is a need for additional pharmaceutical compositions comprising physiologically compatible stabilizers that promote stability of polypeptide components, thereby maintaining their therapeutic effectiveness.

### OBJECTIVES OF THE INVENTION

The main objective of the present invention is to obtain a formulation comprising antibody, buffer and pharmaceutically acceptable excipient(s).

Another objective of the present invention is to obtain a formulation comprising an antibody or fragments thereof, a buffer and lyoprotectants.

Yet another objective of the present invention is to obtain stable formulations of antibody.

### STATEMENT OF THE INVENTION

Accordingly the present invention relates to a Formulation comprising: antibody, buffer and pharmaceutically acceptable excipient(s); a formulation comprising an antibody or fragments thereof, a buffer and lyoprotectants; a stable formulation of antibody comprising: a)about 25 mg/ml to about 250 mg/ml antibody b) buffer providing pH 5 to pH 7.5; a stable formulation of antibody comprising: a)about 100 mg/ml to about 150mg/ml antibody b) buffer providing pH 5 to pH 7.5 c) about 0.001% to about 0.05 % nonionic surfactant; and a stable formulation of antibody comprising: a)about 100 mg/ml to about 150mg/ml antibody b) buffer providing pH 5 to pH 7.5 c) about 0.001% to about 0.05 % nonionic surfactant and c) lyoprotectant and/or cryoprotectant.

### BRIEF DESCRIPTION OF THE ACCOMPANYING FIGURES

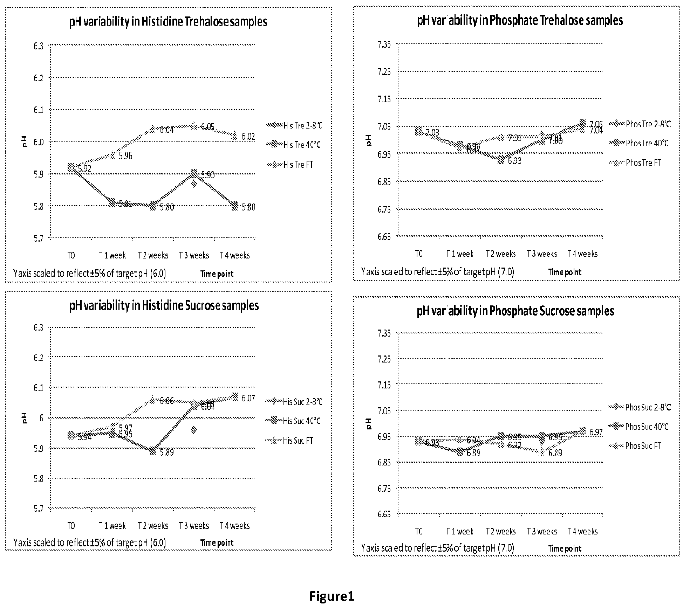

**Figure 1****:** pH variability during the course of study in the different formulations evaluated.

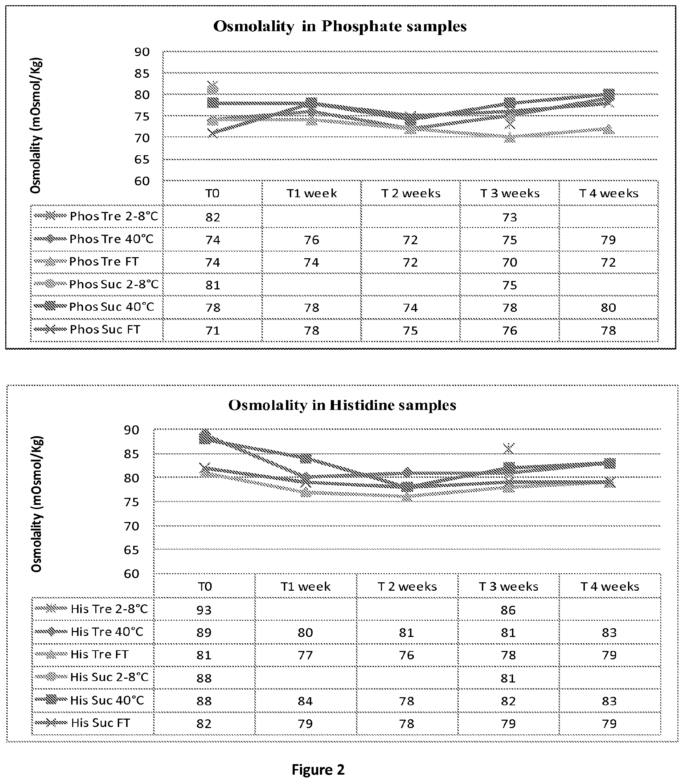

**Figure 2****:** Osmolality values during the course of study in the different formulations evaluated.
**Figure 3****:** Normalized fluorescencegraph showing that the spectrum and lambda max of samples in different formulations incubated at different conditions does not differ.

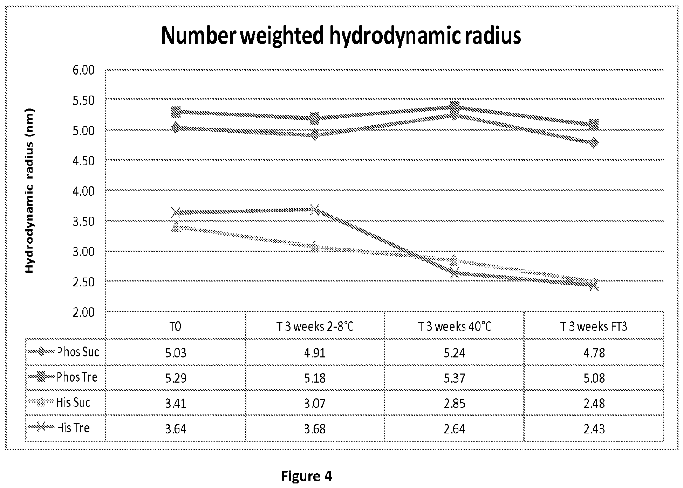

**Figure 4****:** Hydrodynamic radius analysis of the samples.
**Figure 5****:** Charge variant distribution in the samples incubated at 40°C.
**Figure 6****:** Charge variant distribution in the samples incubated at 40°C (histidine trehalose highlighted).

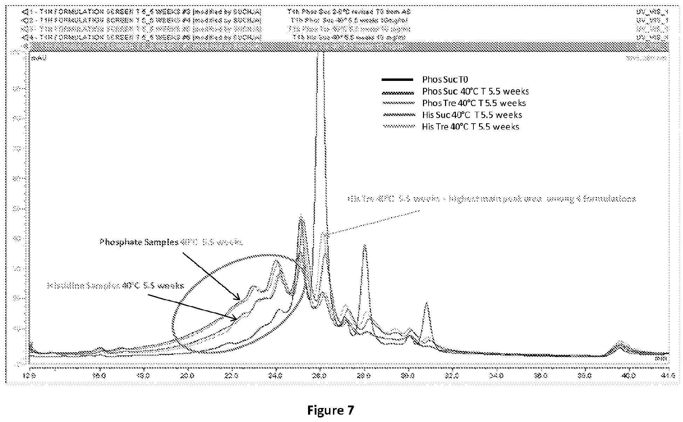

**Figure 7****:** Overlay of Ion exchange chromatography profiles for the phosphate and histidine-based formulations showing the higher acidic variants in the phosphate-based formulations.

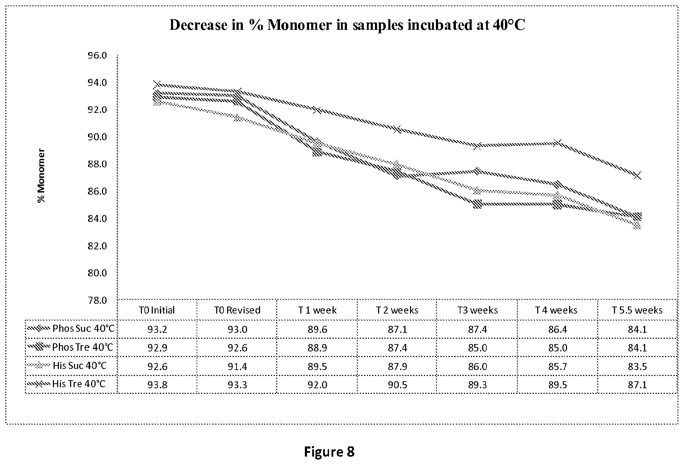

**Figure 8****:** Figure representing the decrease in monomer % in samples incubated at 40°C.

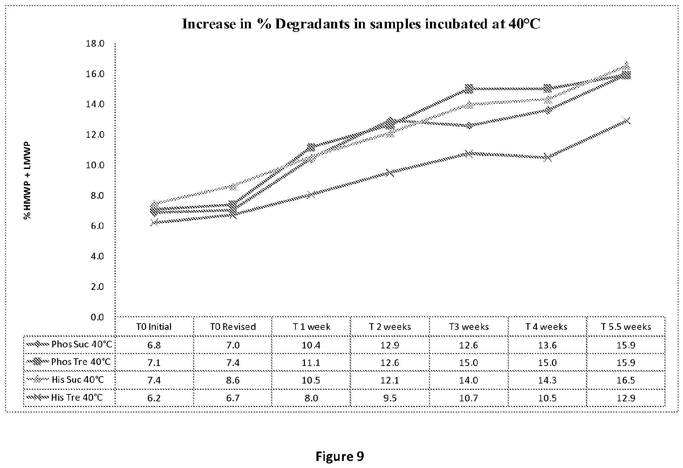

**Figure 9****:** Figure depicting the increase in degradants by SEC (%HMWP + % LMWP) at 40°C.

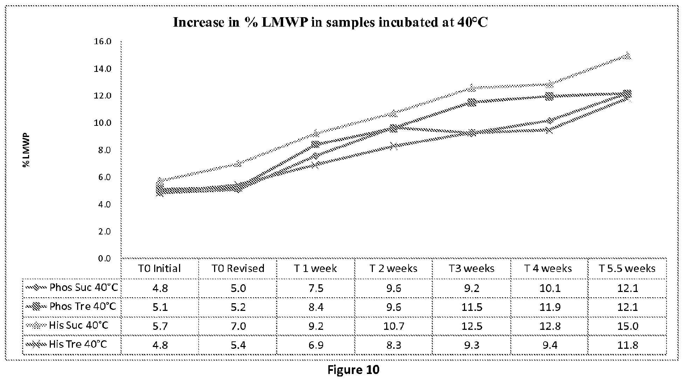

**Figure 10****:** Increase in LMWP in the four formulations tested. All four formulations follow the same trend and the values are very similar except the histidine trehalose formulation has marginally lower fragmentation compared to the others.
**Figure 11****:** Trend for the increase in HMWP in the four formulations tested. The histidine containing formulations exhibit lower aggregation compared to the phosphate samples.
**Figure 12****:** pH variability of Tlh samples in different formulations.
**Figure 13****:** Protein Concentration of the Repeated Freeze-thaw samples.
**Figure 14****:** SEC Profiles of 1 ml samples in phosphate and histidine after repeated Freeze-thaw.
**Figure 15****:** Comparison of SEC profiles of 0.5 ml samples in Phosphate and histidine formulations.
**Figure 16****:** SEC Data of 1 ml samples in both formulations subjected to repeated Freezing and Thawing.
**Figure 17****:** SEC Data of 0.5 ml samples in both formulations subjected to repeated Freezing and thawing.
**Figure 18****:** Overlay of Ion exchange Chromatography profiles of the 1 ml samples showing likely aggregate elution at 40 min in the phosphate samples.
**Figure 19****:** Overlay of the 0.5 ml samples showing aggregate elution in phosphate samples at 40min, but not in Histidine samples.
**Figure 20:** IEX Data of 1 ml samples Subjected to repeated Freezing and thawing in phosphate and Histidine formulations.

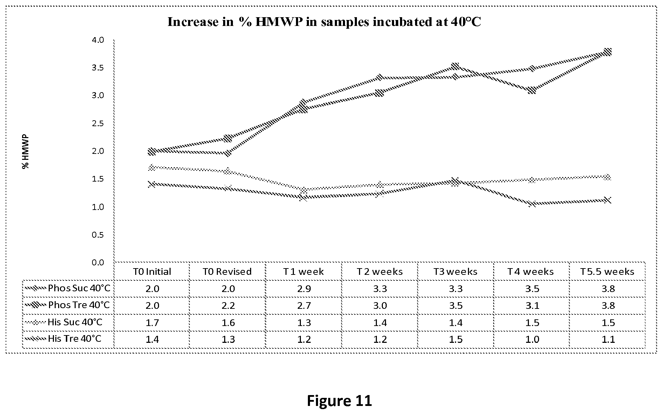

**Figure 11****:** IEX Data of 0.5ml samples Subjected to repeated Freezing and Thawing in Phosphate and histidine formulations.

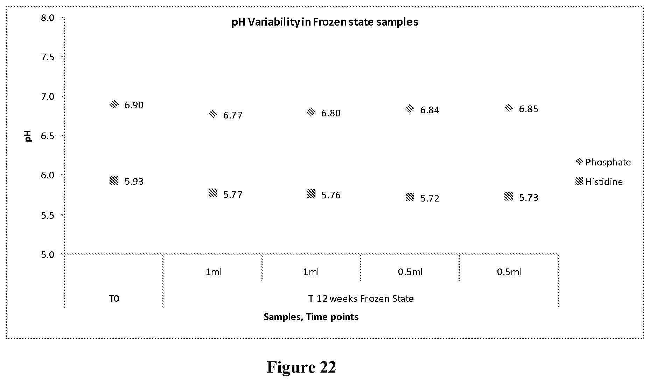

**Figure 22****:** pH variability in frozen state sample.

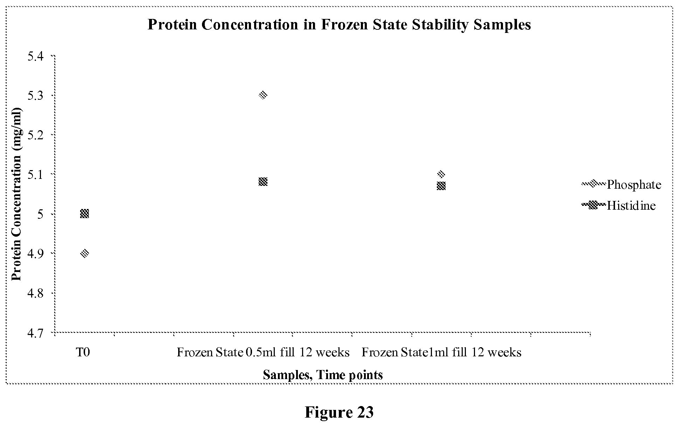

**Figure 23****:** Protein concentration in the frozen state stability samples.

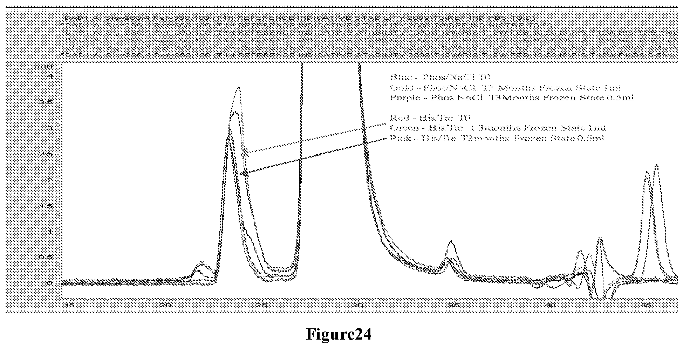

**Figure24****:** SEC Overlays of Frozen state stability showing marginal increase in phosphate aggregation.

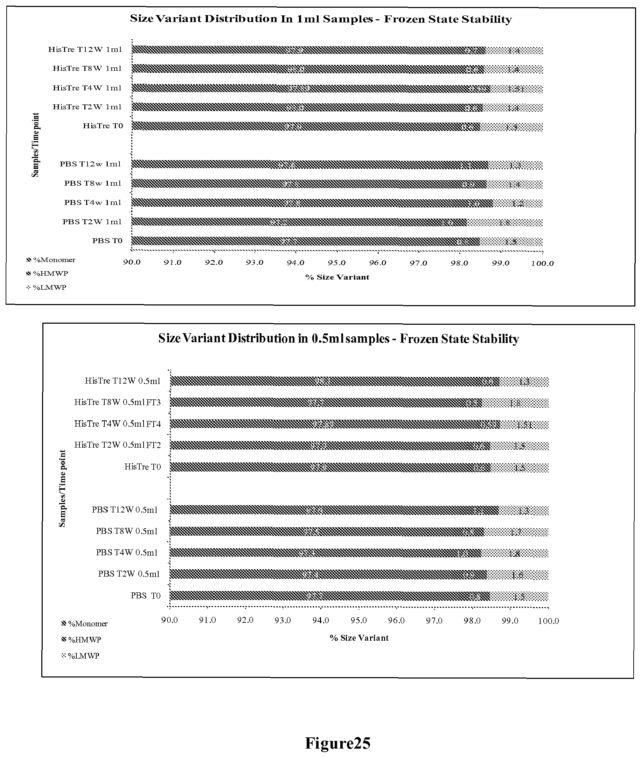

**Figure25****:** SEC data for size variant distribution in frozen state stability samples.

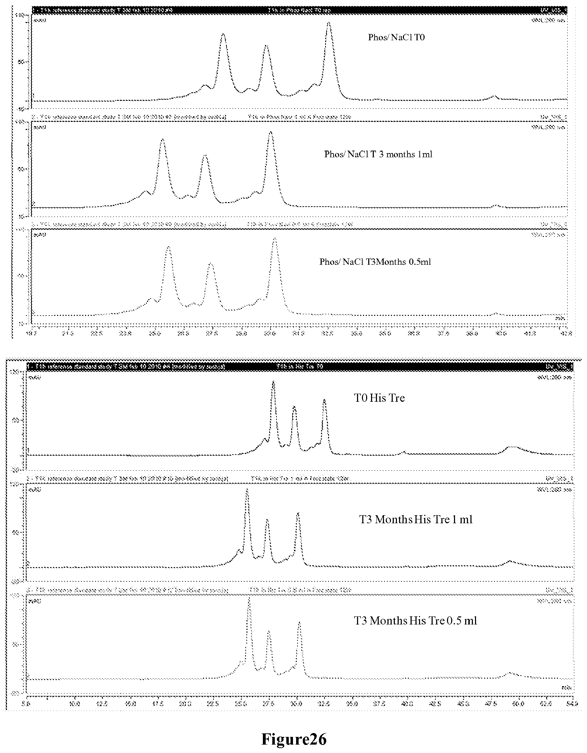

**Figure26****:** IEX Chromatogram Overlays of Frozen state stability samples.

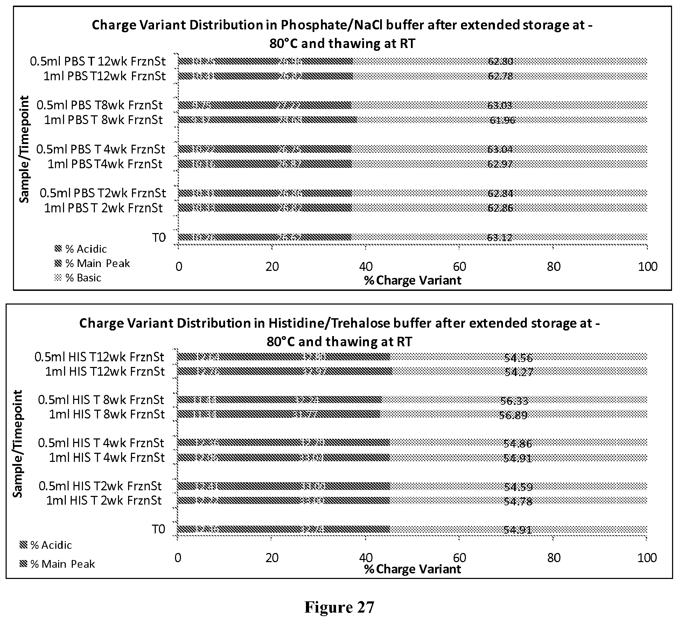

**Figure 27****:** IEX Data for the frozen state stability in phosphate and Histidine.

### DETAILED DESCRIPTION OF THE INVENTION

The present invention relates to a Formulation comprising antibody, buffer and pharmaceutically acceptable excipient(s).

The present invention relates to a formulation comprising an antibody or fragments thereof, a buffer and lyoprotectants.

In an embodiment of the present invention, the buffer is selected from TRIS, phosphates, succinates, citrates, acetates, imidazoles, glycine, L-arginine, histidine and combinations thereof.

In another embodiment of the present invention, the buffer is preferably Histidine.

In another embodiment of the present invention, the lyoprotectants is selected from a polyol or sugars.

In yet another embodiment of the present invention, the lyoprotectants are non-reducing in nature like PEG, glycerol, sucrose, trehalose and mannitol.

In still another embodiment of the present invention, the formulation further comprises cryoprotectants.

In still another embodiment of the present invention, the cryoprotectant is preferably trehalose.

In still another embodiment of the present invention, the formulation further comprises surfactants.

In still another embodiment of the present invention, wherein the surfactants is selected from a group comprising polysorbate 20 and polysorbate 80.

In still another embodiment of the present invention, the formulation further comprises bulking agents.

In still another embodiment of the present invention, wherein the bulking agents are selected from a group comprising glycine and mannitol.

The present invention relates to a stable formulation of antibody comprising: a)about 25 mg/ml to about 250 mg/ml antibody b) buffer providing pH 5 to pH 7.5.

The present invention relates to a stable formulation of antibody comprising: a) about 100 mg/ml to about 150mg/ml antibody b) buffer providing pH 5 to pH 7.5 ; c) about 0.001% to about 0.05 % nonionic surfactant.

The present invention relates to a stable formulation of antibody comprising: a) about 100 mg/ml to about 150mg/ml antibody b) buffer providing pH 5 to pH 7.5 ; c) about 0.001% to about 0.05 % nonionic surfactant; and c) lyoprotectant and/or cryoprotectant.

In an embodiment of the present invention, the formulation is a lyophilized cake or powder.

In an embodiment of the present invention, the formulation is further re-constituted in sterile water for injection or bacteriolytic water for injection.

The primary object of the invention is to provide a stable liquid pharmaceutical composition comprising: an antibody, a buffering species, a polyol and a surfactant.

It has advantageously found that formulation of the composition according to the present invention results in a composition which is stable upon storage. Stable upon storage is taken to mean that the immunoglobulin does not substantially aggregate nor degrade and maintains acceptable levels of in-vitro and in-vivo activity.

Another aspect of the invention relates to a method of preparing the antibody formulation.

The present invention is directed to liquid pharmaceutical compositions comprising an antibody as a therapeutically active component and to methods useful in their preparation. For purposes of the present invention, the term "liquid" with regard to pharmaceutical compositions or formulations is intended to include the term "aqueous". The term "antibody" as used herein encompasses naturally occurring (native), synthetic, and recombinant antibody and proteins, and biologically active variants thereof, as qualified elsewhere herein. By "therapeutically active component" is intended the antibody is specifically incorporated into the composition to bring about a desired, therapeutic response with regard to treatment, prevention, or diagnosis of a disease or condition within a subject when the pharmaceutical composition is administered to that subject.

More particularly, compositions of the invention are stabilized liquid pharmaceutical compositions whose therapeutically active components include an antibody that normally exhibits aggregate formation during storage in liquid pharmaceutical formulations. By "aggregate formation" is intended a physical interaction between the polypeptide molecules that results in formation of oligomers, which may remain soluble, or large visible aggregates that precipitate out of solution. By "during storage" is intended a liquid pharmaceutical composition or formulation once prepared, is not immediately administered to a subject. Rather, following preparation, it is packaged for storage, either in a liquid form, in a frozen state, or in a dried form for later reconstitution into a liquid form or other form suitable for administration to a subject. Aggregate formation by a polypeptide during storage of a liquid pharmaceutical composition can adversely affect biological activity of that polypeptide, resulting in loss of therapeutic efficacy of the pharmaceutical composition. Furthermore, aggregate formation may cause other problems such as blockage of tubing, membranes, or pumps when the polypeptide-containing pharmaceutical composition is administered using an infusion system.

The stabilized liquid pharmaceutical compositions of the invention further comprise an amount of buffering species .These buffering species mainly includes phosphate or Histidine. The buffering species of the formulation is intended to maintain the pH. The pH of the phosphate formulations was set to 7 and that of the histidine formulation was set to 6, since these pHs lies in buffering range of these buffering species.

A stabilizing amount of surfactant is added to the composition of the invention is an amount sufficient to inhibit the formation of aggregates or turbidity in antibody containing compositions. Such aggregate formation can occur upon, for example, long term storage, mechanical agitation, freezing and thawing. Significant inhibition of aggregation or turbidity is observed and the turbidity/aggregate formation is at least 10% less in the antibody containing composition with surfactant than in a comparable formulation that does not contain surfactant, preferably at least 50% less, more preferably at least 70% less, and most preferably at least 90% less. Visual inspection of vials and antibody containing composition with absorbance at 320 nm should be monitored to determine the ability of polysorbate 80 to maintain the protein molecules in solution. The absorbance at 320 nm, arising primarily as a result of scattering of molecules in solution was much more pronounced in samples lacking polysorbate 80.

"Surfactant" as used herein is defined to encompass any detergent that has a hydrophilic region and a hydrophobic region, and includes non-ionic, cationic, anionic and zwitterionic detergents. Suitable surfactants include, for example polyoxyethylene sorbitan monooleate(also known as polysorbate 80 or "TWEEN" 80), polyoxyethylene sorbitan monolaurate (also known as polysorbate 20 or "TWEEN" 20), or N-laurylsarcosine. A non-ionic surfactant is preferable for the formulations described herein. Such non-ionic surfactants can be chosen from the following surfactants such as polyoxamer or polyoxyethylene sorbitan fatty acid esters, for example, polysorbate 20 or polysorbate 80. Polysorbate 80 is preferred for the compositions of this invention. The surfactant may be present in a concentration of 0.01% - 0.5% by weight.

Immunoglobulin subunit polypeptides each comprise a constant region and a variable region. In most species, the heavy chain variable region, or VH domain, and the light chain variable region, or VL domain, combine to form an antigen binding domain comprised of "complementarity determining regions" or CDRs, the portion of an immunoglobulin molecule which specifically contributes to the antigen-binding site for a particular epitope. Generally, heavy and light chains each have three CDRs, which combine to form the antigen binding site of the immunoglobulin. An "antigen binding domain" of an immunoglobulin molecule generally, but not invariably, consists of at least a portion of the variable domain of one heavy chain and at least a portion of the variable domain of one light chain, held together by disulfide bonds. The Fc region is essential to the effector functions of antibodies. The effector functions include initiating complement-dependent cytotoxicity (CDC), initiating phagocytosis and antibody-dependent cell-mediated cytotoxicity (ADCC), and transferring antibodies across cellular barriers by transcytosis. In addition, the Fc region is critical for maintaining the serum half-life of an antibody of class IgG (Ward and Ghetie, Ther. Immunol. 2:77-94 (1995).

A further aspect, the present invention provides an altered antibody or functional fragment selected from Fab, Fc or part thereof.

In a further aspect of the invention provides a pharmaceutical composition comprising an antibody of the present invention or functional fragment thereof together with a pharmaceutically acceptable diluent or carrier.

The composition may include one or more buffering species, one or more polyol, and one or more surfactant.

The composition includes pharmaceutically acceptable carriers. Pharmaceutically accepted carriers include but are not limited to saline, sterile water, phosphate buffered saline, and the like. Other buffering agents, dispersing agents, and inert non-toxic substances suitable for delivery to a patient may be included in the compositions of the present invention. The compositions may be solutions suitable for administration, and are typically sterile and free of undesirable particulate matter.

The buffers used in context of the present invention are preferably Phosphate or histidine. Most preferably the buffers used in the formulations of the instant invention are not limited to acetate, succinate, histidine and phosphate, which may be used as such or in combination.

Desirably the pH of the solution of formulation is in the range 5 to 7.5, and the pH of the solution is preferably in the range 5.5 to 6 with adjustment, if necessary, of the final pH to the desired level.

The polyol used in the context of the present invention are reducing sugar, which play several roles such as stabilizer for antibody, a tonicity modifier and cryoprotectant and lyoprotectant is also included in the formulation. Most preferably the polyol used in the formulation of the instant invention are non - reducing sugar such as sucrose or trehalsoe.

Other pharmaceutically acceptable excipients well known to those skilled in the art may also form a part of the subject compositions. This includes, for example, various bulking agents and wherein bulking agent is selected from glycine or mannitol.

In one embodiment, the sugar component of the formulation (Sucrose and trehalsoe) has a multi-pronged purpose: sugar act as stabilizers for antibody, protecting it from degradation; they are cryo/lyo protectants which protect the antibody during the lyophilization process (which involve both freezing and drying); they are also tonicity modifiers, whose concentration can be adjusted to provide a product that isotonic. Tonicity is of significance in a subcutaneously administered product such as this, since an isotonic product is able to significantly reduce the sting at the site of injection. Sucrose and trehalose were selected for evaluation since they are both non-reducing sugars and have been widely used for stabilizing proteins. The concentration of the sugar was chosen so as to provide an isotonic solution for subcutaneous administration.

In another embodiment of the invention, the results of the formulation screening study indicate that at 2-8°C and when subjected to freezing/thawing no significant differences in HMWP or LMWP were observed between the formulations. However, when incubated at 40°C, the physical stability of the antibody is improved in histidine containing buffers, compared to phosphate-based formulations, especially with respect to aggregation (Figure 11). The T1h sample in histidine trehalose formulation consistently exhibited the highest percentage of monomer and consequently the lowest degradant percentage of all four tested formulations.

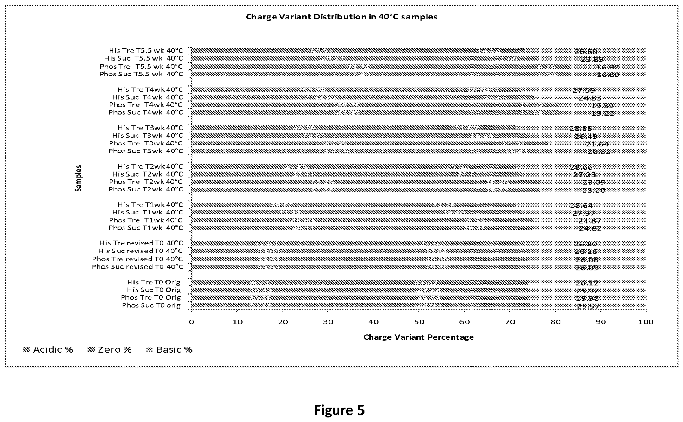

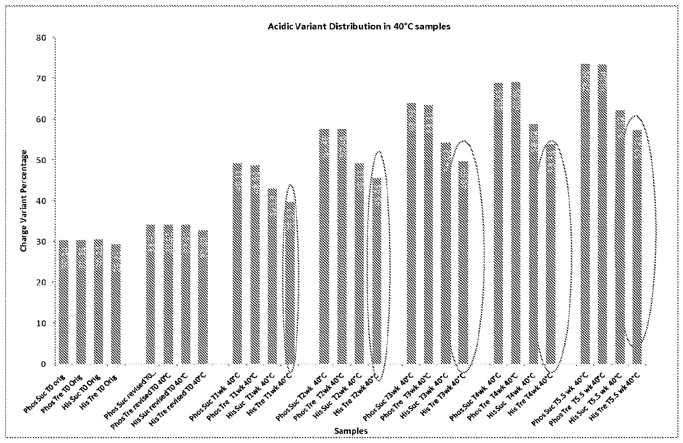

In yet another embodiment of the invention, the charge variant distribution was not altered by incubation of the antibody at 2-8°C or under freezing/thawing. However, incubation of the antibody at 40°C brought forth differences in the extent of stabilization afforded by each formulation. The histidine-based formulations were yet again slower to accumulate acidic variants compared to the phosphate formulations. Among the histidine formulations, histidine trehalose consistently exhibited lower acidic variants, and therefore a higher main peak % compared to histidine sucrose (Figure 5: Charge variant distribution in the samples incubated at 40°C. Figure 6: Charge variant distribution in the samples incubated at 40°C). The potency/biological activity of the antibody is unchanged in any formulation and /or condition, and is comparable to the standard. This conclusion may have been reached since the activity/potency assay is as yet not developed enough to discriminate between the different formulations. This may be an important aspect to consider, in light of the fact that the histidine-based formulations are able to protect the antibody from degradation better than the phosphate formulation

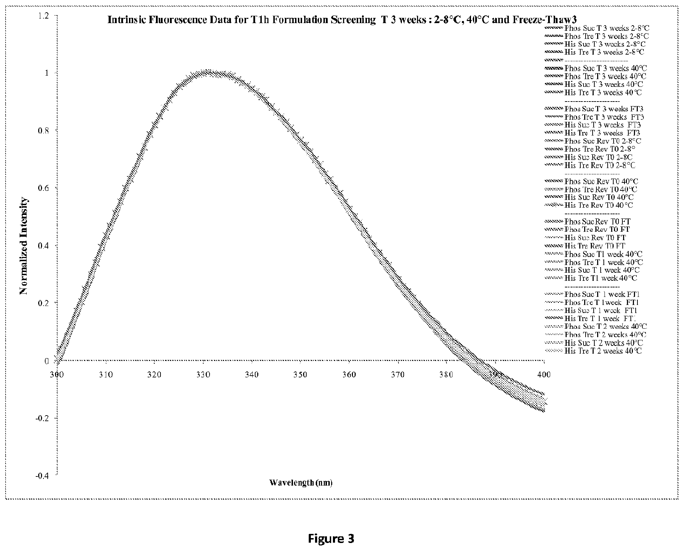

In another embodiment of the invention, the conformational stability of the antibody as assessed by fluorescence spectroscopy and DSC had not undergone a significant change in any of the formulations tested. There is no change in the lambda max (which may point to a change in the 3-D conformation of the antibody) in the fluorescence experiment (Figure 3). Similarly, there is no significant difference in the melting temperatures observed for the T0 samples and the 40°C samples, which indicates that the different domains of the antibody still unfold in much the same way as the start of the study.

The present invention is further defined in the following Examples. It should be understood that these Examples, while indicating preferred embodiments of the invention, are given by way of illustration only. From the above discussion and these Examples, one skilled in the art can ascertain the essential characteristics of this invention, and without departing from the spirit and scope thereof, can make various changes and modifications of the invention to adapt it to various uses and conditions.

The invention will be better understood from the following Examples. However, those of ordinary skill in the art will readily understand that these Examples are merely illustrative of the invention that is defined in the claims that follow thereafter.

The present invention is further elaborated by the following examples and figures. However, these examples should not be construed to limit the scope of the invention.

The following Examples represent preferred embodiments of the present invention.

### EXPERIMENTAL EXAMPLES

A. Formulation containing mAB can be prepared in following ways.

The purified antibody is concentrated by using Tangential Flow filtration (TFF) or UF/DF to the required high concentration and subsequently buffer exchanged into the formulation buffer.

The purified antibody is concentrated to between 20 and 30 mg/ml by using Tangential Flow filtration (TFF) or UF/DF, and subsequently buffer exchanged into the formulation buffer. This sample is then subjected to lyophilization, and once lyophilized, the cake is reconstituted in an appropriate volume of WFI so as to achieve the required final drug product concentration.

The bulk drug substance is lyophilized, and is then dissolved into the formulation buffer to the required concentration.

The antibody is concentrated to the requisite high concentration using column chromatography techniques such as Ion Exchange Chromatography, Affinity chromatography or Hydrophobic Interaction chromatography.

### B.Chemical Stability of the Aqueous Formulation

Samples incubated at 2-8°C and those subjected to freeze-thaw stress did not show any variability by Ion exchange. Differences in the degradation were observed only in the samples incubated at 40°C.

Ion Exchange chromatography of the samples incubated at 40°C clearly shows that the acidic variants increase to a lesser extent in the histidine formulations, compared to the phosphate -based formulations. Among the histidine formulations, the trehalose containing formulation has a lower acidic variant population compared to the histidine sucrose formulation. Histidine trehalose is therefore the superior formulation by Ion Exchange Chromatography

By SEC, the samples incubated at 2-8°C and those that were stressed by freeze-thaw showed little or no difference in degradation pattern among the four formulations evaluated.

However, the samples incubated at 40°C showed differences in the rate at which the monomer decreased, which consequently influenced the accumulation of degradants (HMWP and LMWP) shows that the histidine/trehalose formulation follows a slower rate for the degradation of the monomer, while the other three formulations seem to degrade faster. The same is observed in Figure in the increase in degradants.

A closer observation of the increase in HMWP and LMWP shows that the pattern of increase in LMWP follows a similar trend in all formulations (Figure), and it is in the accumulation of HMWP that the histidine formulations sets themselves apart from the phosphate-based formulations - Figure . At the end of 5.5 weeks, it is the histidine/trehalose containing formulation that has both the highest monomer remaining as well as the lowest % degradants.

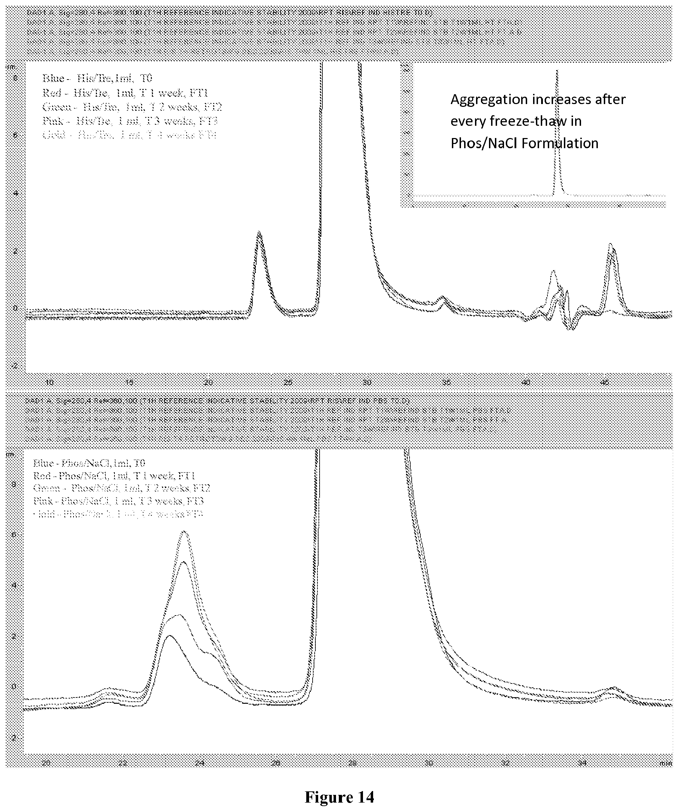

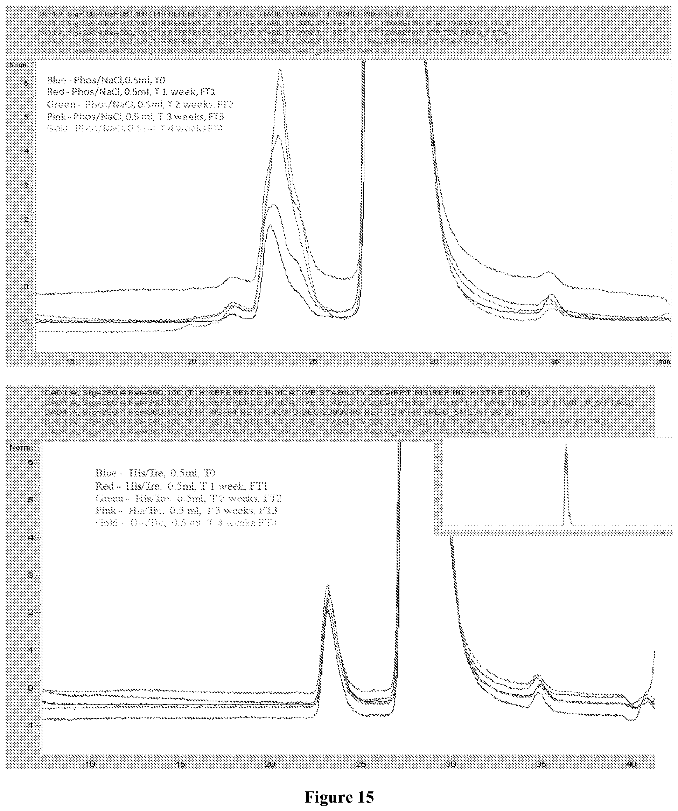

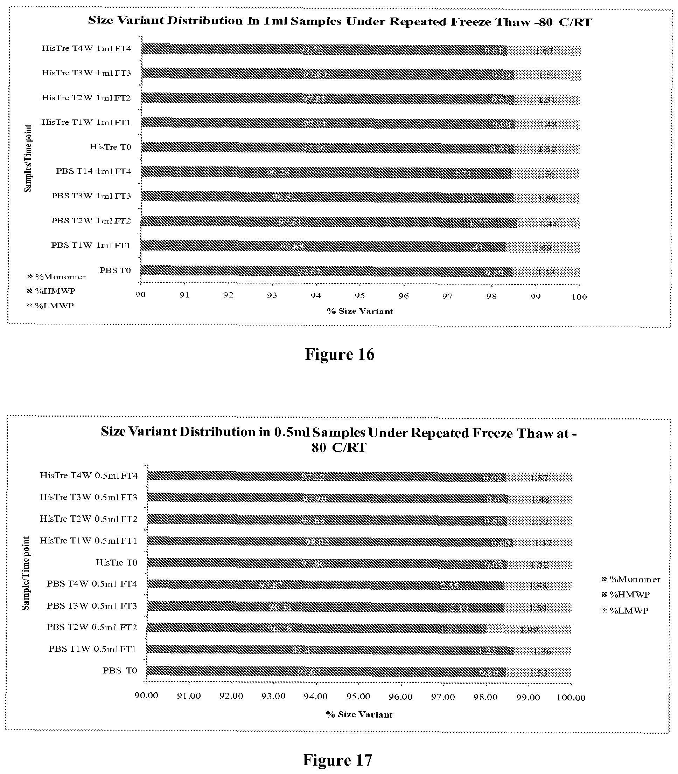

SEC result in figure 14-17 indicate that aggregates increase in phosphate/Nacl sample over four weeks under repeated freeze thawing (-80°C) and there is no observable increase in aggregation in His/Tre formulation over four weeks. The increase in aggregation in aggregation is about 1.75% in the 0.5 ml fill volume sample, and 1.41 % in the 1 ml fill samples.

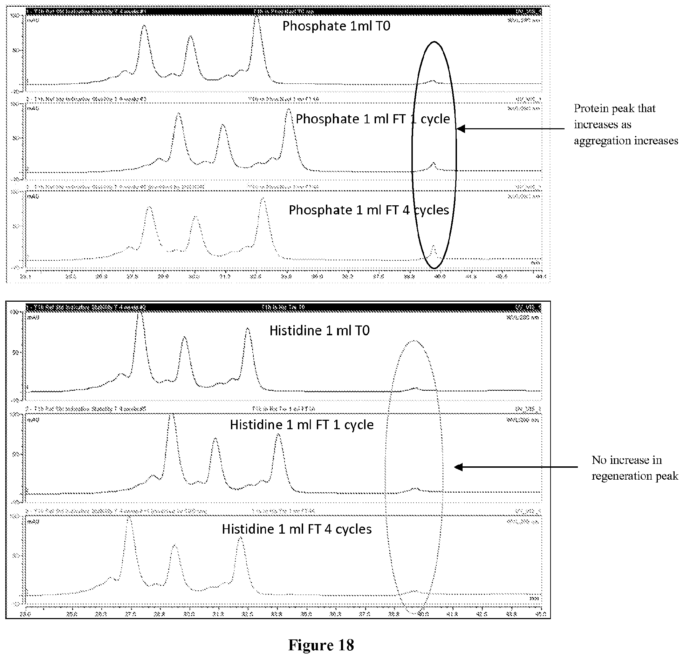

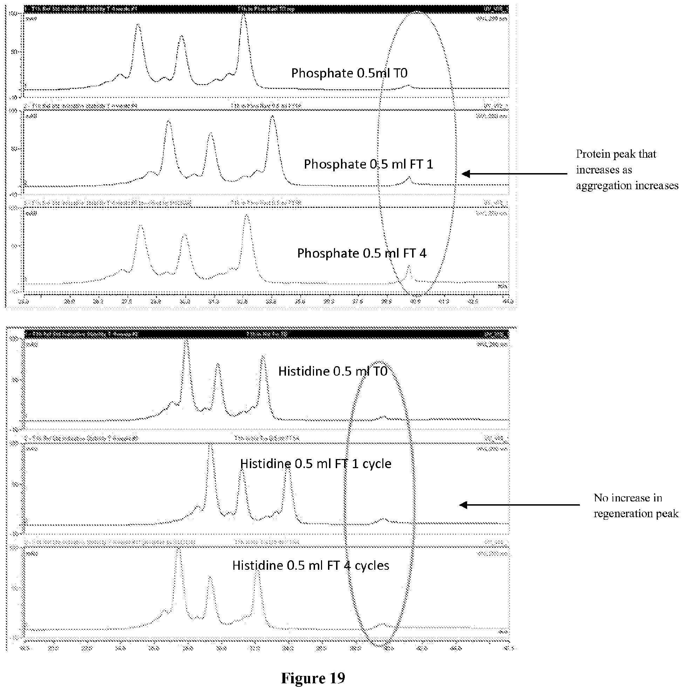

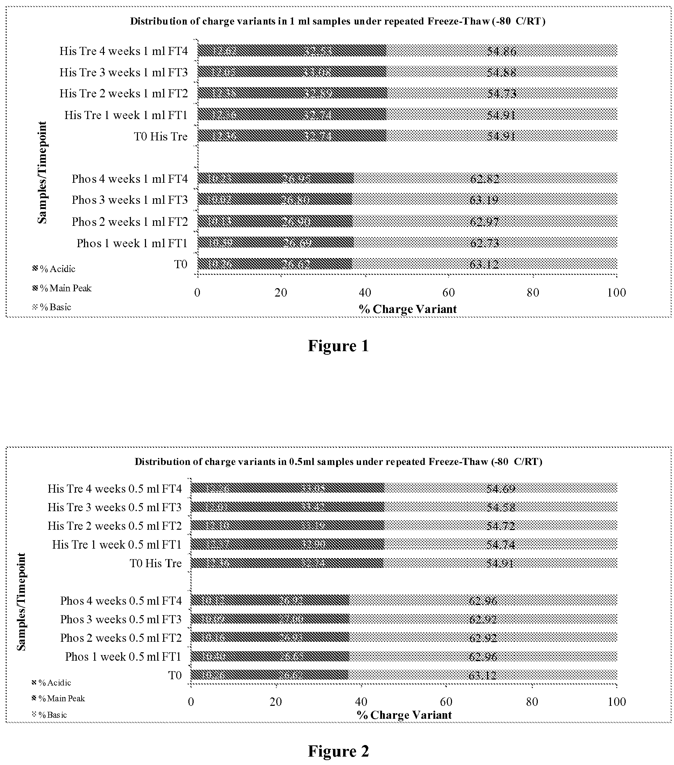

Weak cation exchange chromatography in figure 18- 21 indicate that the charge variant distribution does not change significantly when the mAb is repeatedly frozen (-80°C) and thawed over the four weeks study period in phosphate /Nacl and histidine trehalose formulations.

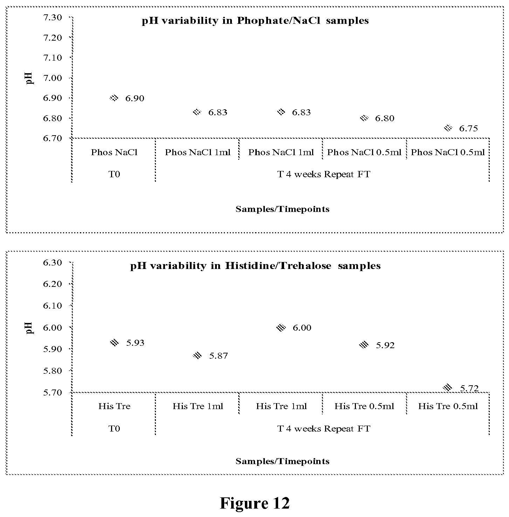

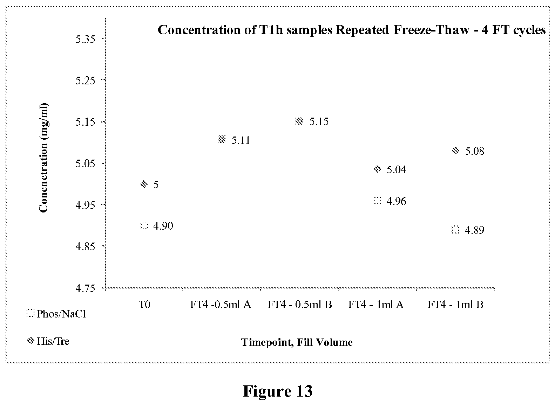

However, in the Phos/Nacl samples, increasing amounts of protein co-elute with the buffer peak at 40 minutes. Other parameters such as pH, concentration do not indicate superiority of one formulation over another as indicated in figure 12-13

The frozen state stability study in figure 24-27 indicate that no difference are observed in the charge variant profile, but that aggregation increases marginally in the Phos/Nacl formulation, and not in the His/Tre formulation. Other parameters such as pH, concentration do not indicate superiority of one formulation over another as indicated in figure 22-23.

The invention further includes the subject matter of the claims of PCT/IB2010/055296 from which this application is derived, the content of which is reproduced below as numbered clauses.
1. A Formulation comprising: antibody, buffer and pharmaceutically acceptable excipient(s).
2. A formulation comprising an antibody or fragments thereof, a buffer and lyoprotectants.
3. The formulation of clause 1 or 2, wherein the buffer is selected from TRIS, phosphates, succinates, citrates, acetates, imidazoles, glycine, L-arginine, histidine and combinations thereof.
4. The formulation of clause 1 or 2, wherein buffer is preferably histidine.
5. The formulation of clause 2, wherein the lyoprotectants is selected from a polyol or sugars.
6. The formulation of clause 5, wherein the lyoprotectants are non-reducing in nature like PEG, glycerol, sucrose, trehalose and mannitol.
7. The formulation of clause 1 or 2, wherein the formulation further comprise cryoprotectants.
8. The formulation of clause 7, wherein the cryoprotectant is preferably trehalose.
9. The formulation of clause 1 or 2, which further comprise surfactants.
10. The formulation of clause 9, wherein the surfactants is selected from a group comprising polysorbate 20 and polysorbate 80.
11. The formulation of clause 1 or 2, wherein the formulation further comprise bulking agents.
12. The formulation of clause 11, wherein the bulking agents is selected from a group comprising glycine and mannitol.
13. A stable formulation of antibody comprising: a) about 25 mg/ml to about 250 mg/ml antibody b) buffer providing pH 5 to pH 7.5.
14. A stable formulation of antibody comprising: a) about 50 mg/ml to about 250mg/ml antibody b) buffer providing pH 5 to pH 7.5 and c) about 0.001% to about 0.05 % nonionic surfactant.
15. A stable formulation of antibody comprising: a) about 100 mg/ml to about 250mg/ml antibody b) buffer providing pH 5 to pH 7.5 ; c) about 0.001% to about 0.05 % nonionic surfactant; and c) lyoprotectant and/or cryoprotectant.
16. The formulation of clause 15, wherein said formulation is a lyophilized cake or powder.
17. The formulation of clause 15, wherein said formulation is further reconstituted in sterile water for injection or bacteriolytic water for injection.

## Claims

1. A histidine-sucrose formulation comprising T1h antibody in an amount from 25 mg/ml to 250 mg/ml antibody, 10 mM to 30 mM histidine buffer, 1 to 15% of sucrose, 0.001 % to 0.05 % surfactant and pH from 5 to 7.5.

2. The histidine-sucrose formulation as claimed in claim 1, wherein the histidine-sucrose formulation further comprises a cryoprotectant.

3. The histidine-sucrose formulation as claimed in claim 1, wherein the histidine-sucrose formulation further comprises a lyoprotectant.

4. The histidine-sucrose formulation as claimed in any of the preceding claims, wherein the surfactant is selected from a group comprising polysorbate 20 and polysorbate 80.

5. The histidine-sucrose formulation as claimed in claim 1, wherein the histidine-sucrose formulation further comprises a bulking agent.

6. The histidine-sucrose formulation as claimed in claim 5, wherein the bulking agents is selected from a group comprising glycine and mannitol.

7. The histidine-sucrose formulation as claimed in any of the preceding claims, wherein the histidine-sucrose formulation is a lyophilized cake or powder.

8. The histidine-sucrose formulation as claimed in claim 7, wherein the histidine-sucrose formulation is further re-constituted in sterile water for injection or bacteriolytic water for injection.

## Patentansprüche

1. Histidin-Sucrose-Formulierung, umfassend T1h-Antikörper in einer Menge von 25 mg/ml bis 250 mg/ml Antikörper, 10 mM bis 30 mM Histidinpuffer, 1 bis 15 % Sucrose, 0,001 % bis 0,05 % Tensid und pH-Wert von 5 bis 7,5.

2. Histidin-Sucrose-Formulierung nach Anspruch 1, wobei die Histidin-Sucrose-Formulierung ferner ein Kryoprotektivum umfasst.

3. Histidin-Sucrose-Formulierung nach Anspruch 1, wobei die Histidin-Sucrose-Formulierung ferner ein Lyoprotektivum umfasst.

4. Histidin-Sucrose-Formulierung nach einem der vorhergehenden Ansprüche, wobei das Tensid aus einer Gruppe ausgewählt ist, die Polysorbat 20 und Polysorbat 80 umfasst.

5. Histidin-Sucrose-Formulierung nach Anspruch 1, wobei die Histidin-Sucrose-Formulierung ferner einen Füllstoff umfasst.

6. Histidin-Sucrose-Formulierung nach Anspruch 5, wobei der Füllstoff aus einer Gruppe ausgewählt ist, die Glycin und Mannit umfasst.

7. Histidin-Sucrose-Formulierung nach einem der vorhergehenden Ansprüche, wobei die Histidin-Sucrose-Formulierung ein gefriergetrockneter Kuchen oder Pulver ist.

8. Histidin-Sucrose-Formulierung nach Anspruch 7, wobei die Histidin-Sucrose-Formulierung ferner in sterilem Wasser für die Injektion oder bakteriolytischem Wasser für die Injektion rekonstituiert wird.

## Revendications

1. Formulation d'histidine-saccharose comprenant l'anticorps T1h en une quantité allant de 25 mg/ml à 250 mg/ml d'anticorps, 10 mM à 30 mM d'un tampon d'histidine, 1 à 15 % de saccharose, 0,001 % à 0,05 % d'un tensioactif et un pH de 5 à 7,5.

2. Formulation d'histidine-saccharose selon la revendication 1, ladite formulation d'histidine-saccharose comprenant en outre un cryoprotecteur.

3. Formulation d'histidine-saccharose selon la revendication 1, ladite formulation d'histidine-saccharose comprenant en outre un lyoprotecteur.

4. Formulation d'histidine-saccharose selon l'une quelconque des revendications précédentes, ledit tensioactif étant choisi dans le groupe comprenant le polysorbate 20 et le polysorbate 80.

5. Formulation d'histidine-saccharose selon la revendication 1, ladite formulation d'histidine-saccharose comprenant en outre un agent de gonflement.

6. Formulation d'histidine-saccharose selon la revendication 5, lesdits agents de gonflement étant choisis dans le groupe comprenant la glycine et le mannitol.

7. Formulation d'histidine-saccharose selon l'une quelconque des revendications précédentes, ladite formulation d'histidine-saccharose étant une poudre ou un gâteau lyophilisé.

8. Formulation d'histidine-saccharose selon la revendication 7, ladite formulation d'histidine-saccharose étant en outre reconstituée dans de l'eau stérile pour injection ou de l'eau bactériolytique pour injection.
